# EUROPEAN PATENT SPECIFICATION

(11) **EP 2 015 672 B1**
(45) Date of publication and mention of the grant of the patent: **27.07.2016**
(21) Application number: 07776150.0
(22) Date of filing: 24.04.2007
(51) Int. Cl.: A61B 5/00

(54) **FIBER OPTIC EVALUATION OF TISSUE MODIFICATION**
FASEROPTISCHE BEURTEILUNG VON GEWEBEMODIFIKATION
ÉVALUATION PAR FIBRE OPTIQUE D'UNE MODIFICATION TISSULAIRE

(30) Priority: 27.04.2006 US 414009
(43) Date of publication of application: 21.01.2009
(73) Proprietor: Lawrence Livermore National Security LLC, Livermore CA 94551-9234 (US); Biosense Webster, Inc., Diamond Bar, CA 91765 (US)
(72) Inventor: SHARAREH, Shiva, G., Laguna Niguel, CA 92677 (US); DEMOS, Stavros, Livermore, CA 94551 (US)
(74) Representative: Small, Gary James
(86) International application number: PCT/US2007/009989
(87) International publication number: WO 2007/127228

(56) References cited:
- EP-A- 0 467 459
- WO-A-93/03672
- WO-A-2004/016155
- WO-A1-2006/055733
- US-A- 5 071 417
- US-A- 5 304 173
- US-A- 5 762 609

## Description

### BACKGROUND OF THE INVENTION

### Field of the Invention

The present invention relates to a medical diagnostic. More particularly, the present invention relates to optical interrogation configurations for investigating tissue modification in real-time during medical procedures.

### Description of Related Art

There are a number of conditions that can be addressed via the destruction of tissue regions to achieve a beneficial result for a patient. Such tissue destruction is typically achieved by subjecting the tissue to conditions outside the environmental profile needed to sustain the tissue alive. As an example, cardiac tissue ablation electrode catheters that can be inserted percutaneously under local anaesthesia into a femoral, brachial, subclavian, or internal jugular vein and positioned in the heart using techniques developed by those skilled in the field is performed to address cardiac arrhythmias, e.g., fibrillation.

In general, ablation systems include an ablation catheter or similar probe having an energy-emitting element. The energy-emitting element delivers energy forming a lesion in the targeted tissue. Typical elements include a microwave ablation element, a cryogenic ablation element, a thermal ablation element, a light-emitting ablation element, an ultrasound transducer, and/or a radio frequency ablation element. The ablation catheter may be adapted to form a variety of lesions such as linear lesions or a circumferential lesion. The element is connected to an energy source that can be varied to control the formation of the lesion.

While various types of ablation catheters for various therapeutic procedures currently exist, catheter ablation of cardiac tissue in particular, is typically performed using radiofrequency energy delivered as a continuous, unmodulated, sinusoidal waveform having a frequency of about 500 kilo-cycles per second. The majority of such systems utilizes the temperature of the ablation electrode to monitor tissue modification, such as lesion formation, and automatically adjusts power output to achieve a targeted electrode temperature. Knowledge of the electrode temperature at a particular ablation site is useful in determining whether the application of radiofrequency produced the desired ablation but it is not sufficient to accurately predict the dimensions of the lesion created, especially its depth.

Spectral diagnostic may also be used for feedback control as disclosed in document US 5 304 173.

Thermal injury is the principal mechanism of tissue destruction during radiofrequency catheter ablation procedures. Elevation of catheter temperature can also result in non-desirable conditions, such as, coagulation of the blood. The development of a coagulum, which can represent a hazard to the patient (i.e., via stroke), results in a rapid increase in impedance which leads to a dramatic decrease in current density, thereby limiting further lesion growth. Moreover, the ablation process can also cause undesirable charring of the tissue and can generate evaporate water in the blood and tissue leading to bursts of microbubbles (i.e., steam pops) during the ablation procedure, which are the result of deposition of energy at a faster than desired rate. Automatic adjustment of power output using closed loop temperature control has been shown to reduce the incidence of coagulum development, steam pops, and undesired charring, which may also facilitate catheter ablation by, for example, reducing the number of times the catheter has to be withdrawn from the body to have a coagulum and charring material removed from the electrode tip.

Despite improvement in the current technologies, no real-time feedback system and method regarding the condition (e.g., the creation of lesions in the lateral and axial dimensions) of the treatment site in addition to the formation of coagulum, steam pops, and charring during catheter ablation within the body is currently available.

Accordingly, a need exists for methods and instrumentation to primarily provide real-time feedback during such procedures as to determine lesion formation, physical dimension, the formation of charred tissue, steam pops, and coagulated blood around a predetermined ablation catheter or endoscopic instrument for any given procedure, medical or otherwise. The present invention is directed to such a need.

### SUMMARY OF THE INVENTION

The present invention is defined in claim 1.

The present invention provides a tool that can be configured with optical fiber arrangements to provide real-time analysis of lesion formations, depth of penetration of a lesion, a cross-sectional area of a lesion in the tissue, recognition of charring, recognition of the formation of coagulum, differentiation of ablated tissue from healthy tissue, and/or recognition of evaporate water in the blood and tissue leading to steam pops.

Accordingly, the present invention provides optical arrangements, capable of directing predetermined spectral radiation and capable of providing received and analyzed spectral information for the determination and quantification of normal or modified tissue. Applications include assessment of tissue parameters during cardiac ablation as well as assessment of tissue properties such as the formation of plaque, artery thickness, and scar tissue.

### BRIEF DESCRIPTION OF THE DRAWINGS

The accompanying drawings, which are incorporated into and constitute a part of the specification, illustrate specific embodiments of the invention and, together with the general description of the invention given above, and the detailed description of the specific embodiments, serve to explain the principles of the invention.
**Fig.1(a)** shows a simplified diagram of a fiber optic evaluation system.
**Fig. 1(b)** shows another example fiber optic evaluation arrangement.
**Fig.1(c)** shows another beneficial fiber optic evaluation arrangement.
**Fig. 2(a)** shows a generic fiber optic implementation within a treatment catheter.
**Fig. 2(b)** shows a beneficial modification of the fiber arrangement within a treatment catheter.
**Fig. 3(a)** shows real-time detection of intensity changes during catheter ablation treatment.
**Fig. 3(b)** shows a real-time monitoring spectrum for 5 different ablation depths.
**Fig. 4** illustrates the relationship between depth and spectral profile using as a marker, the slope of the profile after a linear fit of the profile between 730 nm and 900 nm.
**Fig. 5(a)** illustrates the real-time detection of coagulum formation during catheter ablation treatment from the characteristic changes in the detected spectral profile.
**Fig. 5(b)** illustrates the real-time detection of charring during catheter ablation treatment.

### DETAILED DESCRIPTION OF THE INVENTION

Referring now to the drawings, specific embodiments of the invention are shown. The detailed description of the specific embodiments, together with the general description of the invention, serves to explain the principles of the invention.

Unless otherwise indicated, numbers expressing quantities of ingredients, constituents, reaction conditions and so forth used in the specification and claims are to be understood as being modified by the term "about." Accordingly, unless indicated to the contrary, the numerical parameters set forth in the specification and attached claims are approximations that may vary depending upon the desired properties sought to be obtained by the subject matter presented herein. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the subject matter presented herein are approximations, the numerical values set forth in the specific examples are reported as precisely as possible. Any numerical value, however, inherently contain certain errors necessarily resulting from the standard deviation found in their respective testing measurements.

### General Description

The apparatus and methods, as disclosed herein, allow real-time qualification and quantification of tissue components, often during catheter ablation treatment of predetermined tissue components, such as the heart. By utilizing the disclosed techniques of the present invention, lesion formation, depth of penetration of the lesion, cross-sectional area of the lesion in the tissue, recognition of charring, recognition of the formation of coagulum, differentiation of ablated tissue from healthy tissue, and recognition of evaporate water in the blood and tissue leading to microbubbles (i.e., steam pop formation) is beneficially enabled.

Beneficial ablation catheter embodiments of the present invention are often configured with an optical conduit, i.e., optical fibers or fiber bundles disposed within the catheter from the proximal to about the distal end. The collection and detection system can include any of the optical means for collecting, e.g., refractive and reflective optics, filtering, e.g., notch filters, band-pass filters, edge filters, etc. and/or spectrally dispersing (e.g., using for example, predetermined spectrographs) received polarized and often unpolarized induced spectra so as to capture, and thus best quantify and qualify the spectral information of tissue components often undergoing modification. The detectors themselves often include charged coupled devices (CCDs), (e.g., front and back illuminated CCDs, liquid nitrogen cooled CCDs, on-chip amplification CCDs) but can also include photodiodes, photomultipliers, multi-channel spectral analyzers, two-dimensional array detectors, multi-array detectors, or any equivalent means to provide acquisition, often digitized acquisition, of one or more spectra.

During tissue modification, such as, but not limited to, thermal or cryo tissue ablation, an operator can obtain real-time feedback information about the site undergoing modification. By monitoring the intensity (often up to or greater than a two fold change in peak intensity) of NIR received elastic light scattered spectra between about 600 nm and about 1500 nm, an operator can detect the onset as well as track the progress of tissue ablation.

Moreover, the relative intensity of the red-shifted component of the spectral profile increases as a function of the depth of ablation in time and deposited thermal energy. Thus, the changes in the spectral profile can be used to evaluate the depth of the lesion. In a simplified method of analysis of the changes in a spectral profile, an operator can use the slope of received spectra (i.e., defined by ratios of predetermined spectral bands of received spectra, such as the ratio of the 730 nm over the 910 nm part of the spectrum of received red-shifted spectra) for depth profiling using appropriate calibration methods known to those skilled in the art. Such a beneficial arrangement enables a user to extrapolate ablation depths past the point of directed illumination wavelength penetration depths. Other aspects of the received spectra can be utilized to monitor charring, coagulum, and/or steam pop formation due to observed characteristic changes as shown below in the present invention.

Thus, from such information, operators or automatic software driven directions through closed loop operations can determined the exposure time and/ or terminate a procedure, or increase or decrease the energy delivered to the site as required for a desired effect (e.g., for greater lesion formation at a desired depth), or detect the formation of charring, coagulum, or the formation of steam pops or determine whether an application of ablation energy failed to reach a desired tissue modification.

Accordingly, the present invention provides apparatus for rapid, in-vivo detection and evaluation of modified tissue components. In particular, the present invention provides elastic Near-infrared (NIR) light (i.e., elastic light scattered spectra between about 600 nm and about 1500 nm ) scattering inspection techniques and optical arrangements, often configured with ablation catheter embodiments, as known and utilized by those skilled in the art, to monitor in real-time, human tissue components undergoing tissue modification or for simple probe analysis. Beneficial aspects of utilizing NIR as an analysis means when coupled to probes as discussed herein, include, but are not limited to:
- penetration depths of up to about a few centimeters inside targeted tissue components;
- minimized influence by blood due to low absorption;
- incorporated inexpensive technology;
- no danger to the operator or the patient;
- non-invasively provided information from the surface as well as below the surface of the tissue;
- fiber optic methods that can be easily incorporated in various devises to direct predetermined illumination spectral bands as well as receive real-time feedback from remote locations undergoing treatment.

### Specific Description

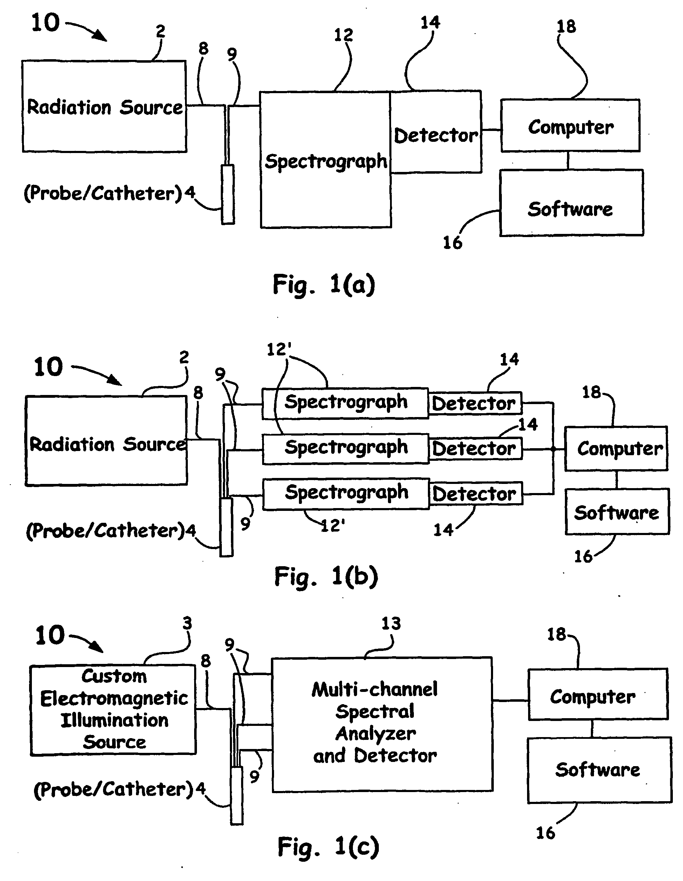

Turning now to the drawings, diagrams that illustrate exemplary basic embodiments of systems are shown in **Figs.1(a)-1(c)****.** Such systems, designated generally by the reference numeral **10,** is most often automated by an analysis means, such as software program **16,** residing on a control analysis means **18** (e.g., a computer, firmware (ROM's, EPROM's) and integrated computational, storage, etc., circuit means, such as, but not limited to, large scale Integrated Circuits LSIC (LSIC), very large scale Integrated Circuits (VLSIC), and field-programmable gate arrays (FPGA's)), which is operably coupled to each component in system **10** by predetermined wireless and or hard communication lines (not shown) such as, USB or RS232 cables. Such software means, firmware means, and other integrated circuit means can provide the filtering, storage and computational manipulations that is desired for the present application. Such communication lines can be constructed and arranged to allow for the exchange of information between analysis means **18** and the system components as shown in **Figs.1(a)-1(c)** to effect operation in a prescribed sequence at the direction of an operator or a predetermined set of programmed instructions to transfer spectral information to analysis means **16** for storage and immediate analysis during operational procedures.

System **10,** also includes an electromagnetic radiation source **2,** as shown in **Fig.1(a)** and **Fig.1(b)****,** for illumination of targeted tissue components. Because the present invention utilizes NIR light scattering and in some arrangements polarized NIR light scattering techniques to determine and quantify tissue modification of, for example, an ablated region of a heart, such a radiation source often includes emission wavelengths of greater than about 250, often a monochromatic laser light source operating at wavelengths of up to about 1500 nm, but most often from about 600 mm to about 970 nm in wavelengths, or from any non-coherent, broadband and/or a coherent source capable of being integrated into the present invention so as to delineate differences in absorption and scattering in human tissue components and to provide mean photon penetration depths of up to about 1 cm. In particular, such sources can include broadband sources (e.g., incandescent lamps, arc lamps, wide-band LEDs), narrow-band spectrally stable light emitting diodes (LEDs), narrow-band fluorescence sources, tunable optical sources (e.g., an optical parametric oscillator, dye lasers, or a Xenon source coupled with a computer controlled monochrometer), narrow-band stable lasers, tripled Nd:Yag systems, etc., all of which are capable of emitting predetermined filtered or otherwise spectral bands to interact with desired tissue components (not shown) so as to induce the desired NIR scattered spectral information.

Such radiation sources **2,** can be configured with probe/catheter **4** via one or more operably coupled optical conduits, e.g., hollow waveguides, light guides, fiber(s) **8,** etc., often large core optical fibers (i.e., multimode fibers) or fibers suitably designed with predetermined fiber indices and dopant profiles, tapered fiber ends and/or special cavity configurations (e.g., bend loss loops), etc. for maintaining polarization properties for predetermined applications, such as when desiring elastic differential light scattering information from a targeted tissue component.

Such differential light scattering techniques that can also be utilized in the present invention is similarly discussed and disclosed in U.S. Patent No. US7016717 B2, titled "Near-Infrared Spectroscopic Tissue Imaging In Medical Applications," by Demos et al. Accordingly, cross-polarization and normalization analysis coupled with inter-spectra operations, such as, but not limited to, subtraction between one or more predetermined received spectra or division between predetermined spectral bands of a received spectra provide information as to the tissue properties resulting from one or more respective probe illumination wavelengths. In addition, the incorporated NIR elastic light scattering intensity measurements of modified tissue components during treatment procedures, often during catheter ablation treatment, using predetermined wavelength cross-polarized light spectrometry, also can provide information for lesion mapping, lesion formation determination and quantification.

As another beneficial arrangement, a custom electromagnetic radiation source(s) **3,** as generically shown in **Fig.1(c)****,** can be configured along with or in substitution of a broadband source, as discussed above, to provide directed desired power levels of at least about 1 µW in one or more spectral bands/wavelengths of up to about 1500 nm, but most often from about 600 nm to about 970 nm in wavelengths, to about the distal end of the probe/catheter **4** via optical fiber(s) **8.** Example custom electromagnetic radiation source(s) **3** can include, but are not limited to, one or more compact substantially coherent commercial diode lasers arranged with the desired spectral bandwidth, power levels, and geometries, for illumination of predetermined tissue components to induce NIR elastic scattered radiation between about 600 nm and about 1500 nm.

Upon illumination of desired tissue components from about the distal end of probe/catheter **4,** via optical fiber(s) **8,** one or more additionally optical fibers **9** (e.g., one or more large core multimode fibers, polarization maintaining fibers, etc.) are additionally configured to collect NIR elastic backscattered information about the distal end of probe/catheter 4 induced by light source **2** or light source 3, as shown in **Figs. 1(a)****-(c).**

It is to be appreciated that the optical fiber embodiments (i.e., fibers shown by reference numerals **8** and **9,** as shown in **Figs.1(a)****-(c)),** and as disclosed herein, can be configured with any probe, such as, a hand-held probe for topical investigation of tissue modification and it is to be noted that such fiber embodiments can be adapted with enhancing optical elements with respect to its ability to deliver and collect light to and from multiple locations in order to accommodate tissue interrogation of catheter positions from about a normal (i.e., 90 degrees) to about a parallel configuration (i.e., 90 degrees from the normal) with the interrogated tissue. Such enhancing optical elements can include, micro-lenses, mirrors, graded-index lenses, diffractive optical elements and other performance enhancing elements as known in the art.

As another beneficial arrangement, optical fiber configurations can be arranged with a probe, such as, for example, any of the rigid scopes utilized during endoscopic surgery and/or any of the flexible scopes generally reserved for diagnostic examinations and biopsies of tubular body cavities and/or structures, e.g., the upper intestinal tract being examined with a gastroscope. Although the optical configurations can be adapted with any of the treatment and/or diagnostic tools currently in the field, most often, however, the optical fiber embodiments entail coupling with any of the surgical ablation devices utilized for treatment of tissue components, such as, tissue components of the heart, prostate, and liver. Exemplary variations of such surgical ablation devices are described in U.S. Patent No. 6,522,930 and discussed in Application Serial No.10/ 260,141 entitled "Fiber-Optic Evaluation of Cardiac Tissue Ablation".

The desired scattered radiation from tissue components as directed by optical conduits (e.g., optical fibers 9) can be filtered through one or more optical components (not shown), such as, edge filters, band-pass filters, polarization filters, prisms, and/or notch filters, etc. Beneficial embodiments, however, can simply include a single spectrograph **12,** as shown in **Fig.1(a)****,** or, one or more spectrographs **12',** as shown in **Fig. 1(b)****,** (three are shown for simplicity), such as when utilizing catheter embodiments that are arranged to provide information to predetermined spectrographs for angular detailed information of a treated site.

Such spectrographs (note: spectrographs, spectrometers, and spectrum analyzers are used interchangeably) often include optical spectrum analyzers, such as, two-dimensional spectrum analyzers, single or single curved line spectrum analyzers, (i.e., a multi-channel spectrum analyzer **13**), to provide, for example, screened cross-section spectroscopic information of a treated or a pre-treatment site. Fourier transform imaging spectrometers or other such devices to allow desired bands and/or polarized components of electromagnetic radiation from tissue components (not shown) can also be used to disperse and analyze received spectra.

A detector **14,** as shown in **Figs.1(a)****,** or a plurality of detectors, as shown in **Fig.1(b)**, (a detector is not shown in **Fig.1(c)** for simplicity) and as discussed above, often include charged coupled devices (CCDs), (e.g., front and back illuminated CCDs, liquid nitrogen cooled CCDs, on-chip amplification CCDs) but can also include photodiodes, photomultipliers, two-dimensional array detectors, a multi-array detector, or any equivalent means of acquisition, often digitized acquisition, of one or more spectra.

The control system software 16, which can be beneficially automated, often includes a graphical user interface (GUI) configured from Visual Basic, MATLAB®, LabVIEW®, Visual C++, or any programmable language or specialized software programming environment to enable ease of operation when performing probe analysis, but more often, probe analysis during catheter ablation treatment of predetermined sites, such as, in predetermined sites of the heart. LabVIEW® and/or MATLAB® in particular, is specifically tailored to the development of instrument control applications and facilitates rapid user interface creation and is particularly beneficial as an application to be utilized as a specialized software embodiment when desired. The received one or more spectra are then captured and stored by analysis means **18** for storage and immediate analysis during operational procedures, which then allows an operator to effect desired changes to, for example, the time of the treatment procedure.

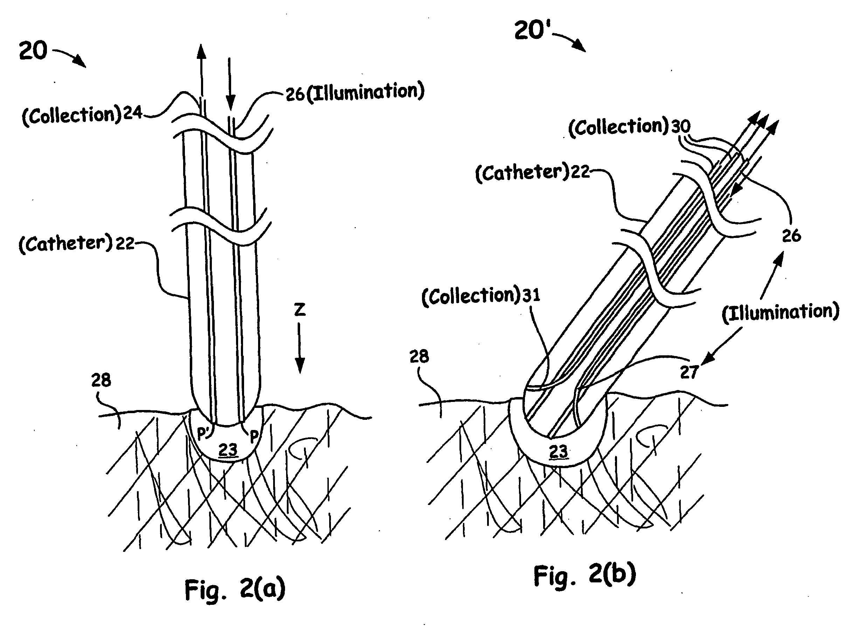

**Fig. 2(a)** shows a basic catheter embodiment of the present invention, generally designated as reference numeral **20,** for real time monitoring of, for example, tissue ablation during treatment of predetermined organs, such as, but not limited to, the liver, prostate, and heart (e.g., a cardiac ablation catheter (e.g., steerable or guidewire catheter embodiments) inserted using, for example, a transseptal or retrograde aortic approach into predetermined sections of the heart to ablate, in some instances, accessory pathways. The optical configurations configured with such a catheter embodiment, or any of the arrangements disclosed herein, can include commercial available optical elements, as known by those of ordinary skill in the art, or custom optical elements to deliver and/or collect predetermined light spectra from multiple locations about the distal end of such catheters.

When utilized with ablation catheter embodiments, catheter **22** can be advanced into the targeted region, wherein a designed ablation element (not shown) of catheter **22** can be energized by means known in the art so as to form, for example, a lesion **23** in the surrounding tissue **28.** When utilized in such a manner, catheter **22** often includes one or more illumination fibers **26** (one shown for simplicity) and one or more collection fibers **24** (again one shown for simplicity), as shown in **Fig. 2(a)****,** running from about the distal end to the proximal end of catheter **22** so as to direct illumination wavelengths and collect desired radiation (as shown with directional arrows) respectively before, during or after application of ablation energy.

As a beneficial embodiment, predetermined illumination radiation of at least about 250 nm and up to about 1500 nm, but most often radiation from about 600 nm to about 970 nm, from one or more illumination fibers **26** configured about the distal end of catheter **22** is directed substantially along the same direction with catheter **22** (direction denoted by the letter **Z** and as shown with a directional arrow). Such directed radiation is received by tissue components, such as normal tissue, non-normal tissue, in addition to modified tissue components, such as lesion **23** along an emission cone angle of illumination fiber(s) **26** or with illumination intensities as produced by adapted enhancing optical elements, such as, but not limited to, micro-lenses, mirrors, graded-index lenses, diffractive optical elements and other fiber performance enhancing elements as known in the art so as to induce NIR elastic scattered light in a backscattered geometry.

Upon such backscattered produced radiation, the one or more collection fibers **24** configured with catheter **22,** receives a predetermined portion of the induced NIR elastic light scattered radiation from probed tissue at a receiving point (denoted as **P'** in **Fig, 2(a)****),** laterally removed from the emitting point of the one or more illumination fibers **26,** (denoted as **P** as shown in **Fig. 2(a)****).** Such induced radiation is then directed by collection fiber(s) **24** to the spectral analysis and detector compartments as illustrated in **Figs.1(a)-****(c)** as detailed above.

The detectors, as shown and discussed above with respect to **Figs.1(a)****-(c),** transforms a photometric signal into an electrical signal. The electrical signal is captured by an electronic circuit (not shown) and is converted to a digital form with conventional analog/ digital converters as known and understood by those skilled in the art. The digital signal is then digitally pre-processed by digital signal processing residing in, for example, analysis means **18,** as shown in **Figs.1(a)****-(c),** and information is stored in memory. The information can be accessed by analysis means **18,** or by one or one or more additional external computing devices (not shown) for further analysis, and presented to users through a graphic user interface via designed or commercial software, as disclosed herein.

A surprising and unexpected result during ablation procedures is the characteristic changes in the received spectra, which enables the detection and determination of deleterious thermal effects (i.e., via intensity and/or characteristic changes in received spectra) resulting from charring, formation of steam pops, and coagulum. The operator can use such information to increase or decrease the energy delivered to the site so as to control the final depth of the lesion while preventing the observed thermal deleterious effects or terminate the ablation procedure altogether.

While such an arrangement, as shown in **Fig. 2(a)** is beneficial, it is to be appreciated that example fibers (i.e., fibers **24** and **26**) used for directing desired radiation components can also be coupled external (not shown) to catheter **22.** In such a non-coupled arrangement, fibers **24** and **26,** are not directly targeting tissue **28** under catheter **22** and thus, such an arrangement is designed to record the presence on ablated tissue (e.g. lesion **23**) as it expands in time outwards from the point of contact with ablation energizing element of catheter **22** and enables ease of operation by not having to overtly modify existing catheter embodiments. As a result, there is a delay time from the point of initiation of ablation to the time that ablation will be detected by the spectroscopic analysis methods when using such an arrangement.

**Fig. 2(b)** shows a variation of the catheter embodiment of **Fig. 2(a)** and is generally designated as reference numeral **20'.** Such an arrangement again can include various probes, such as, but not limited to, a catheter **22** utilized for ablation procedures and modified according to the descriptions presented herein. As illustrated, one or more fibers **30** can again be used for collection while one or more fibers **26** may be used to deliver the illumination. In this novel embodiment, however, one or more additional fibers **27** may be configured with catheter **22** to probe (i.e., illuminate) the tissue, such as a formed or a forming lesion **23** in the case where the catheter is used to ablate the tissue at an angle different than normal to the tissue's **28** surface. The presence of an additional collection fiber **31** not in contact with tissue **28** can also be added by modification to allow catheter embodiments, as shown by example in **Fig. 2(b)****,** to probe the formation of coagulum, steam pops, and/or charring in the area surrounding the catheter that is not in direct contact with tissue **28** and enable evaluation of the orientation of the catheter with respect to the tissue surface. An advanced example arrangement involves a plurality of fibers alternated as illumination and/or collection of scattered light in a predetermined sequence so as to enable even more accurate assessment of the characteristic of ablation and the surrounding catheter environment (formation of coagulum, steam pops, charring, etc.).

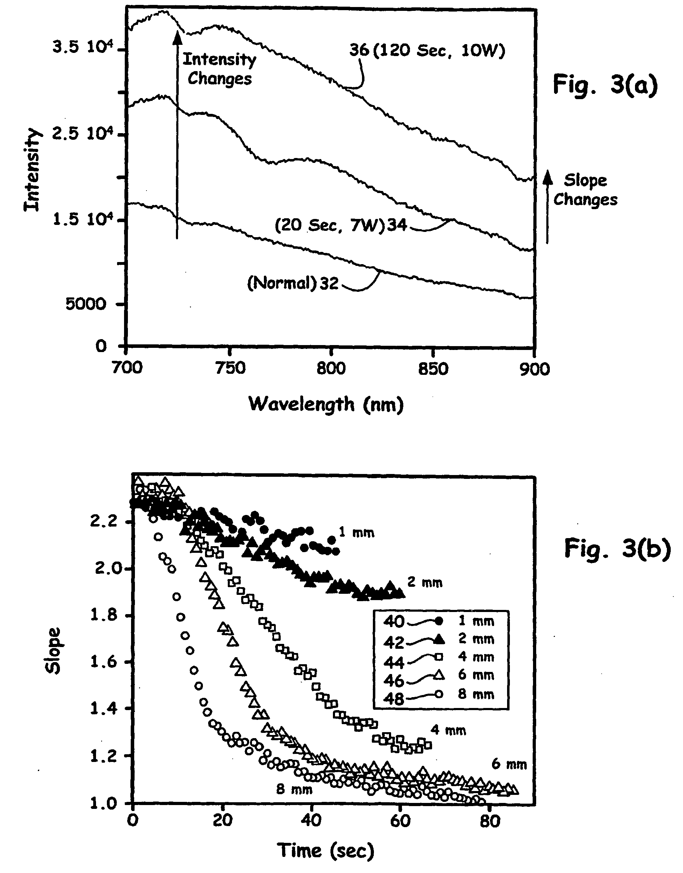

**Fig. 3(a)** shows experimental data of about a two-fold increase (denoted by the directional arrow) in the intensity of the backscattering light during tissue ablation. Such a result is exemplified with spectra from normal tissue **32** exposed to ablation powers of 7W for 20 seconds **34** and subsequently 10W for 120 seconds **36.** Such a change in intensity can be utilized, as one example, to detect steam pop formation (micro bubbles) resulting from heating of the surrounding tissue fluids.

**Fig. 3(a)** also shows a changing slope of the spectral profile towards the longer wavelengths (i.e., at about the 900 run range) (denoted by the shorter directional arrow) due to the ablation exposure times and deposited thermal energy.

**Fig. 3(b)** shows the slope of the spectrum of different sized lesions monitored during ablation lesion formation with different final depths. Thus, **Fig. 3(b)** shows the slope vs. time for 5 different ablations that resulted to lesions having depths of about 1 mm **(40),** 2mm **(42),** 4mm **(44),** 6mm **(46),** and 8 mm **(48).** It is to be appreciated from this experimental data set that the different rates by which the slope is changing depends on the power settings of the catheter. From such data, one can extract the rate of tissue ablation since the slope is related to the depth of the lesion. This can be particular important for deeper lesions where direct measurement of the depth using the fibers may be impossible. More specifically, the measurement of the slope can provide accurate results for lesion depths of up to about 10 mm in human cardiac tissue. However, by measuring the rate of tissue ablation during the initial 6 mm, one can extrapolate the ablation time needed to create lesions of any depth.

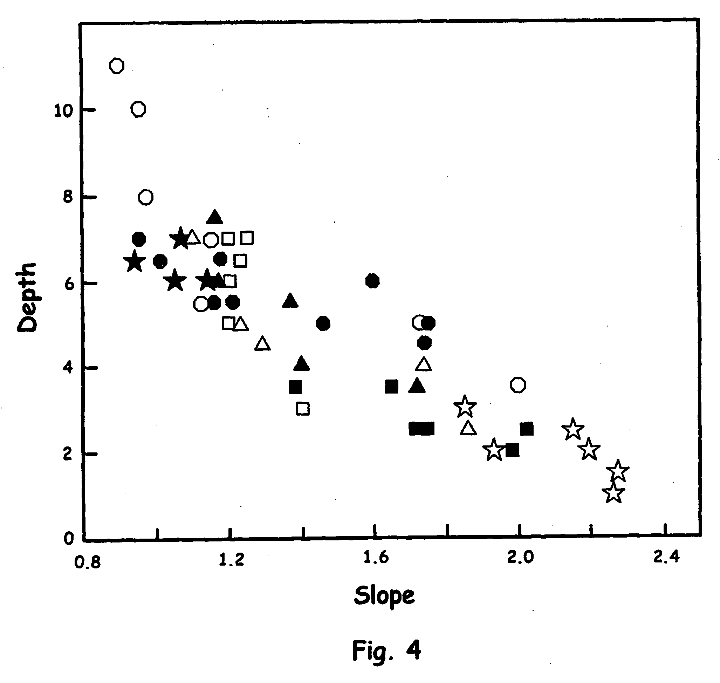

**Fig. 4** illustrates the substantially linear relationship between depth and spectral profile using as a marker, the slope of the profile after a linear fit of the profile between 730 nm and 900 nm. To define an example measured slope, the ratio of the spectral intensity at 730 nm over that at 910 nm is plotted from predetermined spectra received from bovine heart tissue during an ablation procedure for a particular created lesion. Then additional slope values for different lesions created using different ablation times and power settings resulting in different lesion depths is added to the overall plot, as shown in **Fig. 4****.** Accordingly, **Fig. 4** summarizes experimental results showing the depth of the ablated tissue and the corresponding slope of the accompanying spectral profile. These results clearly indicate an almost linear relationship between these two parameters for lesion depths up to about 6 mm.

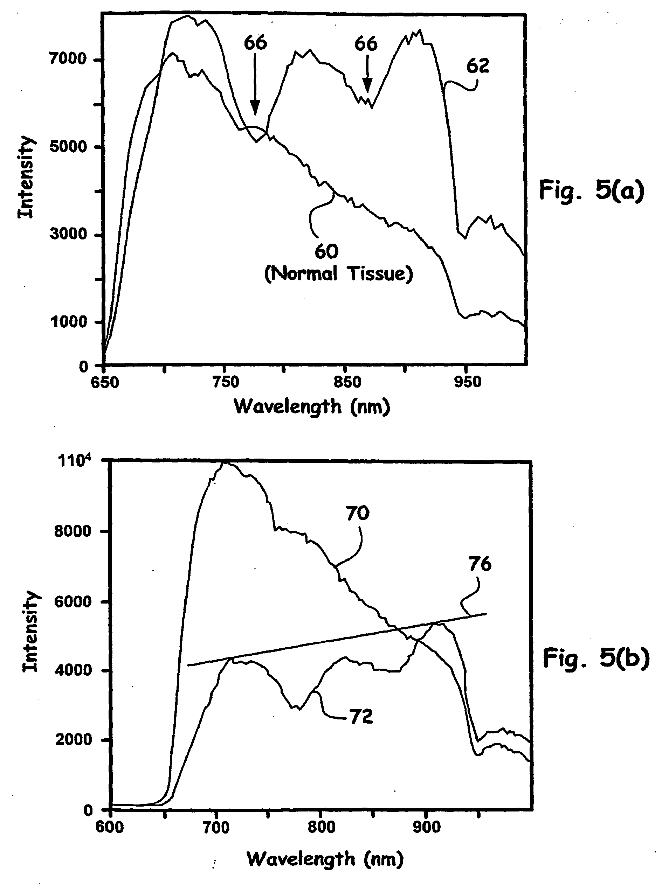

**Fig. 5(a)** illustrates the real-time detection of coagulum formation during catheter ablation treatment from the characteristic changes in the detected spectral profile while **Fig. 5(b)** illustrates the real-time detection of charring during catheter ablation treatment. Thus, **Fig. 5(a)** shows a normal tissue spectrum **60** and the presence of two spectral dips **66** in a received spectrum **62,** indicating the presence of two absorption peaks associated with the presence of coagulum. **Fig. 5(b)** shows a spectrum of normal tissue **70** and a subsequent spectrum **72** in the presence of charring. From the results of Fig. **5(b)** charring tends to exhibit intensities of the scattered light at 730 nm that is lower to that at 910 nm (i.e., for the spectral calibration used during this experiment). This leads to an example value of the estimated slope of less than 1. The absolute values of the slope shown above are somewhat arbitrary. This comes from the fact that the recorded spectra have not been corrected for instrument response nor for the spectral profile of the white light used for illumination. Therefore, although all trends and qualitative behaviors describe above are valid, the absolute values of the slopes and the relative intensities of the spectra at different wavelengths need to be adjusted to take into account instrument response and spectrum of input illumination light

Accordingly, the present invention utilizes primarily NIR light scattering **to provide information about predetermined tissue properties prior to as well as during certain predetermined therapeutic procedures.** In particular, with respect to ablation procedures, the present disclosure can provide information with regards to **lesion formation, depth of penetration of the lesion, cross-sectional area of the lesion in the tissue, recognition of charring, recognition of the formation of coagulum, differentiation of ablated tissue from healthy, diseased, and/or abnormal tissue, and recognition of evaporate water in the blood and tissue leading to microbubbles (i.e., steam pop formation) is beneficially enabled.**

Applicants are providing this description, which includes drawings and examples of specific embodiments, to give a broad representation of the invention. Various changes and modifications within the scope of the invention will become apparent to those skilled in the art from this description and by practice of the invention. The scope of the invention is not intended to be limited to the particular forms disclosed and the invention covers all modifications, equivalents, and alternatives falling within the scope of the invention as defined by the claims.

## Claims

1. An apparatus for assessing tissue components, comprising:
an ablation catheter;
one or more interrogation radiation sources operable at predetermined wavelengths;
a plurality of optical fibers disposed within said ablation catheter for directing radiation from said interrogation radiation sources to one or more targeted tissue components and additionally adapted for receiving and directing an induced backscattered radiation resulting from said one or more interrogated and targeted tissue components;
a device adapted to record one or more spectra of said induced backscattered radiation from said tissue components; and
means for analyzing one or more spectral changes of said spectra so as to enable in real-time, recognition of charring, recognition of the formation of coagulum, and/ or recognition of evaporate water in the blood and tissue leading to steam pops
wherein at least one of said plurality of fibers is an illumination fiber directed substantially along the same direction as the catheter, and at least one of said plurality of optical fibers is arranged at a predetermined angle thereto so as to probe the formation of coagulum, steam pops, and/or charring in the area surrounding a region undergoing ablation and enable evaluation of the orientation of the catheter with respect to the tissue surface.

2. The apparatus of claim 1, wherein a relative increase in intensity and a red-shifted component of said recorded spectra is utilized to real-time monitor one or more modified tissue components.

3. The apparatus of claim 1, wherein a rate of said spectral changes enables extrapolation of an ablation depth of up to about 1 cm or of the normal tissue.

4. The apparatus of claim 1, wherein said interrogation radiation source is operable at emission wavelengths of between about 600 nm and about 970 nm.

5. The apparatus of claim 1, wherein said interrogation radiation source comprises at least one source selected from: broadband sources, narrow-band spectrally stable light emitting diodes (LEDs), narrow-band fluorescence sources, laser sources, and tunable optical sources.

6. The apparatus of claim 1, wherein said backscattered radiation comprises a spectral region between about 600 nm and about 1500 nm.

7. The apparatus of claim 1, wherein said plurality of optical fibers comprise polarization maintaining fibers.

8. The apparatus of claim 1, wherein said plurality of optical fibers comprises a plurality of fibers alternated as illumination and/ or collection fibers of directed and scattered radiation in a predetermined sequence.

9. The apparatus of claim 1, wherein said detector device comprises at least one detector devices selected from: a charged coupled devices (CCDs), photodiodes, photomultipliers, spectral analyzers, two- dimensional array detectors, and multi-array detectors.

10. The apparatus of claim 1, wherein said analyzing means further comprises at least one device selected from: a computer, a firmware, a CPU, a graphical user interface, a software program, and a field-programmable gate array.

11. The apparatus of claim 1, wherein said treatment and/ or diagnostic tool is configured such that it can be positioned up to about 90 degrees from the normal with respect to said one or more targeted tissue components.

## Patentansprüche

1. Vorrichtung zum Beurteilen von Gewebekomponenten, umfassend:
einen Ablationskatheter;
eine oder mehrere Untersuchungsstrahlungsquellen, die bei vorbestimmten Wellenlängen funktionsfähig sind;
eine Vielzahl von optischen Fasern, die in dem Ablationskatheter angeordnet sind, zum Richten von Strahlung aus den Untersuchungsstrahlungsquellen auf ein oder mehrere angezielte Gewebekomponenten, und die zusätzlich dafür ausgelegt sind, eine induzierte rückgestreute Strahlung, die von dem einen oder den mehreren untersuchten und angezielten Gewebekomponenten stammt, zu empfangen und zu leiten;
eine Vorrichtung, die dafür ausgelegt ist, ein oder mehrere Spektren der induzierten rückgestreuten Strahlung von den Gewebekomponenten aufzuzeichnen; und
Mittel zum Analysieren einer oder mehrerer spektraler Veränderungen der Spektren, um zu ermöglichen, in Echtzeit Verkohlung zu erkennen, die Entstehung von Koagulum zu erkennen und/oder das Verdampfen von Wasser in dem Blut und Gewebe, das zu Aufplatzen von Dampf führt, zu erkennen;
wobei wenigstens eine aus der Vielzahl von Fasern eine Beleuchtungsfaser ist, die im Wesentlichen entlang der gleichen Richtung wie der Katheter gerichtet ist, und wenigstens eine aus der Vielzahl von optischen Fasern in einem vorbestimmten Winkel dazu angeordnet ist, um die Entstehung von Koagulum, Aufplatzen von Dampf und/oder Verkohlung in dem Gebiet zu erfassen, das einen Bereich umgibt, der Ablation erfährt, und die Auswertung der Orientierung des Katheters bezogen auf die Gewebeoberfläche zu ermöglichen.

2. Vorrichtung gemäß Anspruch 1, wobei eine relative Zunahme der Intensität und eine rotverschobene Komponente des aufgezeichneten Spektrums verwendet wird, um eine oder mehrere modifizierte Gewebekomponenten in Echtzeit zu überwachen.

3. Vorrichtung gemäß Anspruch 1, wobei die Rate der spektralen Veränderungen die Extrapolation einer Ablationstiefe bis zu etwa 1 cm oder des Normalgewebes ermöglicht.

4. Vorrichtung gemäß Anspruch 1, wobei die Untersuchungsstrahlungsquelle bei Emissionswellenlängen zwischen etwa 600 nm und etwa 970 nm funktionsfähig ist.

5. Vorrichtung gemäß Anspruch 1, wobei die Untersuchungsstrahlungsquelle wenigstens eine Qualle ausgewählt aus: Breitbandquellen, schmalbandigen spektral stabilen lichtemittierenden Dioden (LEDs), Schmalband-Fluoreszenzquellen, Laserquellen und einstellbaren optischen Quellen umfasst.

6. Vorrichtung gemäß Anspruch 1, wobei die rückgestreute Strahlung einen Spektralbereich zwischen etwa 600 nm und etwa 1500 nm umfasst.

7. Vorrichtung gemäß Anspruch 1, wobei die Vielzahl von optischen Fasern polarisationserhaltende Fasern umfasst.

8. Vorrichtung gemäß Anspruch 1, wobei die Vielzahl von optischen Fasern eine Vielzahl von Fasern abwechselnd als Beleuchtungs- und/oder Sammelfasern von gerichteter und gestreuter Strahlung in einer vorbestimmten Reihenfolge umfasst.

9. Vorrichtung gemäß Anspruch 1, wobei die Detektorvorrichtung wenigstens eine Detektorvorrichtung ausgewählt aus: Charge-coupled Devices (CCDs), Photodioden, Photovervielfachern, Spektralanalysatoren, zweidimensionalen Arraydetektoren und Multiarraydetektoren umfasst.

10. Vorrichtung gemäß Anspruch 1, wobei das Analysatormittel ferner wenigstens eine Vorrichtung ausgewählt aus: einem Computer, einer Firmware, einer CPU, einer graphischen Benutzerschnittstelle, einem Softwareprogramm und einem feldprogrammierbaren Gate-Array umfasst.

11. Vorrichtung gemäß Anspruch 1, wobei das Behandlungs- und/oder diagnostische Werkzeug so gestaltet ist, dass es bis zu etwa 90 Grad von der Normalen bezogen auf die eine oder mehreren angezielten Gewebekomponenten positioniert werden kann.

## Revendications

1. Appareil destiné à évaluer des composants tissulaires, comprenant :
un cathéter d'ablation ;
une ou plusieurs sources de rayonnement d'interrogation utilisables à des longueurs d'onde prédéterminées ;
une pluralité de fibres optiques disposées à l'intérieur dudit cathéter d'ablation pour diriger le rayonnement provenant desdites sources de rayonnement d'interrogation jusqu'à un ou plusieurs composants tissulaires ciblés et également adaptées pour recevoir et diriger un rayonnement rétrodiffusé induit résultant dudit ou desdits composants tissulaires interrogés et ciblés ;
un dispositif adapté pour enregistrer un ou plusieurs spectres dudit rayonnement rétrodiffusé induit provenant desdits composants tissulaires ; et
un moyen pour analyser une ou plusieurs modifications spectrales desdits spectres de manière à permettre, en temps réel, la reconnaissance d'une carbonisation, la reconnaissance de la formation de caillots, et/ou la reconnaissance d'eau évaporée dans le sang et le tissu conduisant à des explosions de vapeur,
dans lequel au moins une fibre de ladite pluralité de fibres est une fibre d'éclairage dirigée sensiblement dans la même direction que le cathéter, et au moins une fibre de ladite pluralité de fibres optiques est disposée à un angle prédéterminé par rapport à celui-ci de manière à sonder la formation de caillots, des explosions de vapeur et/ou une carbonisation dans la zone entourant une région subissant une ablation et permettre une évaluation de l'orientation du cathéter par rapport à la surface tissulaire.

2. Appareil de la revendication 1, dans lequel une augmentation relative d'intensité et une composante décalée vers le rouge desdits spectres enregistrés sont utilisées pour surveiller en temps réel un ou plusieurs composants tissulaires modifiés.

3. Appareil de la revendication 1, dans lequel une vitesse desdites modifications spectrales permet l'extrapolation d'une profondeur d'ablation allant jusqu'à environ 1 cm ou du tissu normal.

4. Appareil de la revendication 1, dans lequel ladite source de rayonnement d'interrogation est utilisable à des longueurs d'onde d'émission comprises entre environ 600 nm et environ 970 nm.

5. Appareil de la revendication 1, dans lequel ladite source de rayonnement d'interrogation comprend au moins une source choisie parmi : les sources à large bande, les diodes électroluminescentes (DEL) spectralement stables à bande étroite, les sources de fluorescence à bande étroite, les sources laser, et les sources optiques accordables.

6. Appareil de la revendication 1, dans lequel ledit rayonnement rétrodiffusé comprend une région spectrale comprise entre environ 600 nm et environ 1500 nm.

7. Appareil de la revendication 1, dans lequel ladite pluralité de fibres optiques comprend des fibres maintenant la polarisation.

8. Appareil de la revendication 1, dans lequel ladite pluralité de fibres optiques comprend une pluralité de fibres alternées sous forme de fibres d'éclairage et/ou de collecte de rayonnement dirigé et diffusé selon une séquence prédéterminée.

9. Appareil de la revendication 1, dans lequel ledit dispositif de détection comprend au moins un dispositif de détection choisi parmi : les dispositifs à couplage de charge (CCD), les photodiodes, les photomultiplicateurs, les analyseurs spectraux, les détecteurs à réseau bidimensionnel, et les détecteurs à réseaux multiples.

10. Appareil de la revendication 1, dans lequel ledit moyen d'analyse comprend en outre au moins un dispositif choisi parmi : un ordinateur, un microprogramme, une unité centrale, une interface utilisateur graphique, un programme informatique, et un réseau prédiffusé programmable par l'utilisateur.

11. Appareil de la revendication 1, dans lequel ledit outil de traitement et/ou de diagnostic est configuré de telle sorte qu'il peut être positionné jusqu'à environ 90 degrés par rapport à la normale à un ou plusieurs composants tissulaires ciblés.
